(19) Europäisches Patentamt
European Patent Office
Office européen des brevets

(11) Veröffentlichungsnummer: **0 507 003 A2**

# (12) EUROPÄISCHE PATENTANMELDUNG

(21) Anmeldenummer: **91121889.9**

(22) Anmeldetag: **20.12.91**

(51) Int. Cl.5: **C07C 233/39**, C07C 219/10, C11D 1/46, C11D 1/52, A61K 7/075

(30) Priorität: **03.04.91 DE 4110663**

(43) Veröffentlichungstag der Anmeldung: **07.10.92 Patentblatt 92/41**

(84) Benannte Vertragsstaaten: **BE CH DE DK ES FR GB GR IT LI LU NL**

(71) Anmelder: **REWO Chemische Werke GmbH**
**Industriegebiet West**
**W-6497 Steinau an der Strasse(DE)**

(72) Erfinder: **Die Erfinder haben auf ihre Nennung verzichtet**

(54) **Neue Ammoniumverbindungen, Verfahren zu ihrer Herstellung und ihre Verwendung als Reinigungsmittel, kosmetischer Rohstoff und Weichmacher, insbesondere als Weichspülmittel für Gewebe.**

(57) Ammoniumverbindungen und ihre Verwendung als Textilweichspülmittel, bestehend aus Mischungen quatärer, gegebenenfalls Estergruppen enthaltender Ammoniumverbindungen, welche hergestellt werden durch Umsetzung von Aminen mit dimerisierten Fettsäuren und anschließende Quaternisierung oder Protonierung mit anorganischen oder organischen Säuren.

EP 0 507 003 A2

Die vorliegende Erfindung betrifft neue Ammoniumverbindungen, Verfahren zu ihrer Herstellung und ihre Verwendung als Reinigungsmittel, kosmetischer Rohstoff und Weichmacher, insbesondere als Weichspülmittel für Gewebe.

Beim Waschen von Textilien werden bekannterweise im letzten Waschgang sogenannte Weichspüler eingesetzt, um die Verhärtung der Gewebe durch das Trocknen zu vermindern und den Griff der behandelten Textilien angenehm zu beeinflussen.

Als Weichspüler werden üblicherweise kationische Verbindungen verwandt, beispielsweise quatäre Ammoniumverbindungen, die neben langkettigen Alkylresten auch Ester- oder Amidgruppen enthalten können. Vorteilhafterweise verwendet man auch Mischungen verschiedener weichmachender Komponenten, die in Form wässriger Dispersionen dem Spülbad zugegeben werden.

Obgleich diese kationischen Verbindungen wirksame Weichmacher bei der Verwendung im letzten Spülbad darstellen, weisen sie beim Gebrauch immer noch gewisse Nachteile auf.

Einer der Nachteile derartiger Mittel ist, daß sich die weichmachenden Komponenten nicht in kaltem Wasser dispergieren lassen; weiterhin ist das Rücknetzvermögen der mit ihnen behandelten Textilien noch nicht befriedigend.

Unter Rücknetzvermögen wird im allgemeinen die Aufnahme von Feuchtigkeit durch die Faser verstanden. Ein mangelhaftes Rücknetzvermögen wirkt sich aber dort nachteilig aus, wo größere Mengen Feuchtigkeit von der Hautoberfläche aufgenommen werden sollen, z. B. bei Hand- oder Badetüchern sowie bei Leib- oder Bettwäsche.

Aufgabe der vorliegenden Erfindung war es, die obengenannten Nachteile herkömmlicher Weichspülformulierungen zu überwinden und hochkonzentrierte Wäscheweichspülmittel bereitzustellen, die neben guter biologischer Abbaubarkeit und weichem Griff eine wesentlich verbesserte Dispergierbarkeit bzw. Löslichkeit auch in kaltem Wasser und ein verbessertes Rücknetzvermögen aufweisen.

Überraschenderweise wurde gefunden, das Textilweichspülmittel, bestehend aus Mischungen quatärer gegebenenfalls Estergruppen enthaltender Ammoniumverbindungen, die durch Quaternisierung oder durch Protonierung mit anorganischen oder organischen Säuren herstellbar sind, nicht nur diese Anforderungen erfüllen, sondern selbst in hohen Konzentrationen bis zu ca. 30 Gew.-% in kaltem Wassser klar löslich sind.

Gegenstand der Erfindung sind Ammoniumverbindungen der allgemeinen Formel (1)

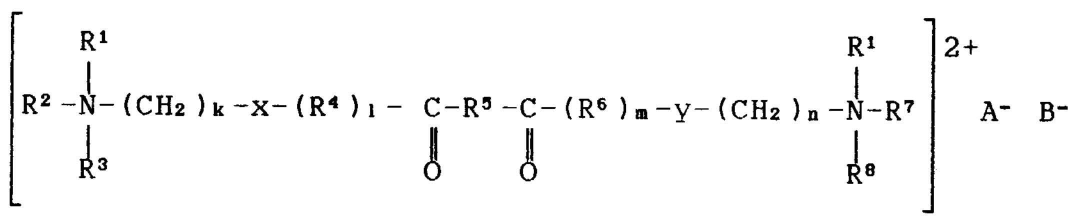

(1)

mit der Bedeutung von

$R^1$ = gleich oder verschieden H, $-CH_3$, $-C_2H_5$, $-(C_3H_5O)_{C1}H$, $-(C_2H_4O)_{C2}H$

$R^2$, $R^3$, $R^7$, $R^8$ = gleich oder verschieden ein geradkettiger verzweigter Alkyl-oder Alkylenrest mit 1 - 18 C-Atomen

$R^4$, $R^6$ = $-(CH_2-CHR^9-O)_a-(CH_2-CHR^{10}-O)b-$ mit $R^9 R^{10}$ = H oder $-CH_3$, a, b = gleich oder verschieden 0 - 10

$R^5$ = Rest der dimerisierten Fettsäure

x, y = O und/oder NH

$A^-B^-$ = gleich oder verschieden das Anion einer wasserlöslichen ein- oder mehrwertigen anorganischen oder organischen Säure wie Methylschwefelsäure, Ethylschwefelsäure, einer Halogenwasserstoffsäure, Phosphorsäure, Ameisensäure, Essigsäure, Oxalsäure, Glykolsäure, Zitronensäure, Weinsäure, Äpfelsäure und insbesondere Salzsäure und Milchsäure sein können und die Gesamtzahl oder negativen Ladungen im Molekül gleich der positiven ist

k, n = gleich oder verschieden 2 - 4

$C_1$, $C_2$ = gleich oder verschieden 1 - 5

l, m = gleich oder verschieden 0 oder 1, mit l = O, wenn x = NH und m = O wenn y = NH.

Ein weiterer Gegenstand der Erfindung sind wässrige Weichspülmittel, enthaltend

A) 5 bis 30 Gewichts-% mindestens einer der Verbindungen der allgemeinen Formel 1

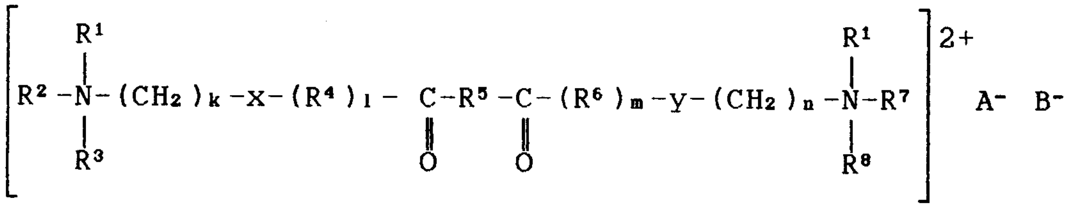

(1)

mit der Bedeutung von

| | |
|---|---|
| $R^1$ | = gleich oder verschieden H/-$CH_3$, -$C_2H_5$ -$(C_3H_5O)_{C1}H$, -$(C_2H_4O)_{C2}H$ |
| $R^2$, $R^3$, $R^7$, $R^8$ | = gleich oder verschieden ein geradkettiger verzweigter Alkyl-oder Alkylenrest mit 1 - 18 C-Atomen |
| $R^4$, $R^6$ | = -$(CH_2$-$CHR^9$-$O)_a$-$(CH_2$-$CHR^{10}$-$O)_b$- mit $R^9 R^{10}$ = H oder -$CH_3$, a, b = gleich oder verschieden 0 - 10 |
| $R^5$ | = Rest der dimerisierten Fettsäure |
| x, y | = O und/oder NH |
| $A^-B^-$ | = gleich oder verschieden das Anion einer wasserlöslichen ein- oder mehrwertigen anorganischen oder organischen Säure wie Methylschwefelsäure, Ethylschwefelsäure, einer Halogenwasserstoffsäure, Phosphorsäure, Ameisensäure, Essigsäure, Oxalsäure, Glykolsäure, Zitronensäure, Weinsäure, Äpfelsäure und insbesondere Salzsäure und Milchsäure sein können und die Gesamtzahl oder negativen Ladungen im Molekül gleich der positiven ist |
| k, n | = gleich oder verschieden 2 - 4 |
| $C_1$, $C_2$ | = gleich oder verschieden 1 - 5 |
| l, m | = gleich oder verschieden 0 oder 1, mit l = O, wenn x = NH und m = O wenn y = NH |

und ergänzend auf 100 Gew.-%

B) Wasser, Farbstoffe, Parfüm, Verdickungsmittel und übliche Hilfs- und Zusatzstoffe.

Erfindungsgemäß werden vorzugsweise Verbindungen der allgemeinen Formel 1 eingesetzt, in denen $R^1$ gleich oder verschieden H oder -$CH_3$ ist, $R^{2,3,7,8}$ der Methylrest,
$R^5$ der Rest der dimerisierten Fettsäure mit einem Dimergehalt von ≧80 Gew.-% ist
k, n = 2 oder 3, l und m = 0 und $A^-$und $B^-$ gleich oder verschieden der Lactatrest oder der Methosulfatrest ist.

Die erfindungsgemäß mitverwendeten Verbindungen der allgemeinen Formel 1 werden nach an sich bekannten Verfahren hergestellt, wobei im allgemeinen das prim./tert. Amin oder das Alkanolamin in Gegenwart saurer Katalysatoren wie Methansulfonsäure oder hypophosphoriger Säure unter Stickstoff bei Temperaturen von 140 - 200 °C verestert bzw. amidiert und das entstandene Kondensat kontinuierlich, gegebenenfalls im Endstadium bei Unterdruck, abdestilliert wird. Anstelle der Fettsäuren können auch deren Ester verwendet werden.

Die zur Herstellung der erfindungsgemäßen Verbindungen mitverwendeten dimeren Fettsäuren sind die handelsüblichen Produkte, welche durch Polymerisation von gesättigten oder ungesättigten natürlichen oder synthetischen einbasischen aliphatischen Fettsäuren mit 16 - 22 Kohlenstoffatomen nach bekannten Methoden hergestellt werden (vgl.-US-PS 2 482 761, US-PS 3 256 304). Typische im Handel erhältliche dimere Fettsäuren haben etwa folgende Zusammensetzung:

| | |
|---|---|
| Monomere Säuren | 0 - 15 Gew.-% |
| dimere Säuren | 60 - 95 Gew.-% |
| tri- und höherpolymerisierte Säuren | 1 - 35 Gew.-% |

wobei der Gehalt je nach Herkunft der Monomeren, des Polymerisationsverfahrens sowie des Aufbereitungsprozesses innerhalb dieser Grenzen schwanken kann.

Die eingesetzte dimere Fettsäure kann auch in hydrierter Form vorliegen.

Der Gehalt an dimerer Säure kann durch allgemein bekannte Destillationsverfahren bis zu 100 Gew.-% erhöht werden.

Es wird bestimmt nach den bekannten Verfahren der Gas-Liquid-Chromatography (GLC).

Bei Verwendung von destillierter dimerer Fettsäure werden Produkte mit verbesserter Farbzahl erhalten. Andererseits ist die Verwendung von handelsüblicher technischer polymerisierter Fettsäure zur Herstellung durchaus möglich. Bei der Verwendung von technischer dimerer Fettsäure sei darauf hingewiesen, daß bei höheren Gehalten an trimerer Fettsäure Produkte mit schlechterer Farbzahl und höheren Viskositäten erhalten werden können. Dies hängt von dem jeweiligen Gehalt an dimerer und monomerer Fettsäure der polymerisierten Fettsäure ab, und die gewünschten Werte können durch wenige einfache orientierende Versuche, wie sie zur handwerklichen Alltagsroutine des Durchschnittsfachmanns gehören, festgestellt werden. Falls gewünscht, können erfindungsgemäß auch die bei der Destillation der dimerisierten Fettsäuren anfallenden Destillationsrückstände allein oder in Mischung mit handelsüblichen dimeren Fettsäuren mitverwendet werden. Diese Destillationsrückstände haben im allgemeinen etwa die folgende Zusammensetzung:

| | |
|---|---|
| Monomere Säuren: | 0 - 1 Gew.-% |
| Dimere Säuren: | 15 - 25 Gew.-% |
| Tri- und höherpolymere Säuren: | 75 - 85 Gew.-% |

Bevorzugt wird jedoch destillierte dimere Fettsäure mit einem Gehalt an dimerer Fettsäure von 80 - 96 % verwendet.

Die erfindungsgemäß mitverwendeten Alkanolamine bzw. primäre und tertiäre Amingruppen im Molekül enthaltenden Amine sind handelsübliche Produkte der allgemeinen Formeln 2a und 2b

$R^2,R^3$-N-$(CH_2)_2$-OH (2a)

$R^2,R^3$-N-$(CH_2)_3$-$NH_2$ (2b)

in welchen $R^2$ und $R^3$ die oben angegebene Bedeutung haben, vorzugsweise aber Alkylreste mit 1 - 4 C-Atomen haben und erfindungsgemäß bevorzugt Methyl- oder Ethylreste sind.

Die Verbindungen der Formel 2a können nach an sich bekannten Verfahren alkoxyliert, das heißt erfindungsgemäß bevorzugt ethoxyliert werden. Im allgemeinen wird dabei so verfahren, daß man die Amine in einem Druckreaktor bei 120 - 160 °C, gegebenenfalls in Gegenwart basischer, insbesondere alkalischer Katalysatoren bei 1 - 4 bar mit einer dem gewünschten Alkoxylierungsgrad entsprechenden Menge an Alkylenoxid, erfindungsgemäß bevorzugt sind Ethylenoxid und Propylenoxid oder deren Mischungen, abreagiert.

Erhalten werden Verbindungen der allgemeinen Formel 2c

$R^2,R^3$-N-$(CH_2)_2$-O-$(CH_2$-$CH_2$-$O)_a$-$(CH_2CHR^{10}$-$0)_b$H mit a, b = 0-10 (2c)

wobei a + b ≧1 ist.

Die mitverwendeten erfindungsgemäßen Verbindungen können in an sich bekannter Weise mit organischen oder anorganischen Säuren protoniert werden. Vorzugsweise verfährt man dabei so, daß zu der Reaktionsmischung die wässrige Protonensäure gegebenenfalls unter Mitverwendung von Lösungsmitteln wie beispielsweise kurzkettigen Alkoholen, unter intensivem Rühren zugegeben wird.

Beispiele für die mitverwendeten organischen Säuren sind Ameisensäure, Essigsäure, Methylschwefelsäure, Ethylschwefelsäure, insbesondere Milchsäure, Äpfelsäure, Weinsäure, Zitronensäure, Oxalsäure, Glykolsäure; anorganische Säuren wie Halogenwasserstoffsäuren, insbesondere Salzsäure, Schwefelsäure, Phosphorsäure. Erfindungsgemäß bevorzugt werden Milchsäure und Salzsäure.

Beispiele für die mitverwendeten Quaternierungsmittel sind kurzkettige Dialkylphosphate und -sulfate wie insbesondere Dimethylsulfat, Dimethylcarbonat, Diethylsulfat, Dimethylphosphat, Diethylphosphat, kurzkettige Halogenkohlenwasserstoffe, insbesondere Methylchlorid.

Die Quaternierung erfolgt nach den bekannten Verfahren. Erfindungsgemäß wird vorzugsweise so verfahren, daß die geschmolzene Substanz, gegebenenfalls unter Mitverwendung eines Lösungsmittels, vorzugsweise kurzkettige Alkohole wie iso-Propanol, bei 60 - 90 °C mit equimolaren Mengen des Quaternierungsmittels unter Rühren, gegebenenfalls unter Druck, versetzt wird und die Vervollständigung der Reaktion durch Kontrolle der Gesamtaminzahl (GAZ) überwacht wird.

Durch Verwendung der erfindungsgemäßen Verbindungen können Weichspülmittel hergestellt werden, welche bis zu Konzentrationen von ca. 25 - 30 Gew.-%, bezogen auf Gesamtmischung, auch in kaltem Wasser klare Lösungen ergeben und Textilmaterialien, besonders solchen aus natürlicher und regenerierter Cellulose sowie Wolle und Frottee neben einem angenehm weichen Griff ein hervorragendes Rücknetzvermögen verleihen.

Die erfindungsgemäßen Weichspülmittel werden neben den üblichen Textilmaterialien daher insbesondere dort eingesetzt, wo größere Mengen Nässe und Feuchtigkeit innerhalb kurzer Zeit von der Körperoberfläche entfernt werden sollen wie bei Hand- oder Badetüchern. Aber auch dort, wo Feuchtigkeit innerhalb größerer Zeitspannen direkt von der Haut aufgenommen werden muß, wie bei Leib- oder Bettwäsche, sind die erfindungsgemäßen Weichspülmittel erfolgreich einsetzbar.

Die Herstellung der Weichspülmittelkonzentrate erfolgt durch Lösen der jeweiligen Einzelkomponenten in Wasser. Hierbei können die auf diesem Gebiet üblichen Verfahrensweisen für Dispersionen angewandt werden, wobei die hier erforderliche Erwärmung sowohl des Wassers als auch der Aktivsubstanz unterbleiben kann.

Üblicherweise wird dabei so vorgegangen, daß in das vorgelegte Wasser bei Raumtemperatur unter gutem Rühren erst die Farbstofflösung, dann die gegebenenfalls erforderliche Antischaumemulsion und dann die erfindungsgemäße Aktivsubstanz zugegeben wird. Soweit erforderlich, können die üblichen Hilfs- und Zusatzstoffe und Parfümöle ebenfalls zugegeben werden. Es sind dies insbesondere Komplexbildner, optische Aufheller, Farb- und Duftstoffe, Elektrolyte und höhermolekulare Etherverbindungen zur Viskositätsregulierung, geringe Mengen organischer Lösungsmittel und - soweit sie das Rücknetzvermögen nicht nachteilig beeinflussen - übliche Tenside. Falls erforderlich, wird mit den auf diesem Gebiet üblichen anorganischen bzw. organischen Säuren wie insbesondere HCl, $H_3PO_4$, $H_2SO_4$, Milchsäure oder Citronensäure auf pH-Werte von ca. 3 - 5 eingestellt.

Wie die zum bekannten Stand der Technik gehörenden Weichspülmittel werden die erfindungsgemäßen Weichspüler im Anschluß an den eigentlichen Waschvorgang im letzten Spülgang zugegeben. Die Anwendungskonzentration liegt nach dem Verdünnen mit Wasser je nach Anwendungsgebiet im Bereich von 0,1 - 10 g Weichspülmittel pro Liter Behandlungsflotte.

Die erhaltenen Konzentrate mit einem Aktivstoffgehalt von bis zu ca. 30 Gew.-%, bezogen auf die Gesamtrezeptur, sind niedrigviskose klare Lösungen, welche neben den oben angeführten Vorteilen sich ausgesprochen problemlos im Spülwasser der Waschmaschinen verteilen.

Zur Beurteilung des Griffs wird das Textilgut aus Wolle, Baumwolle, Polyester/Baumwolle 50 : 3 und Polyester ca. 10 Minuten lang mit einer Flotte aus Leitungswasser (ca. 9 °dH und einer Temperatur von 15 - 20 °C) und erfindungsgemäßer Dispersion behandelt. Die Konzentration der erfindungsgemäßen Verbindungen in der Flotte beträgt 0,025 Gew.-%, bezogen auf Gesamtaktivsubstanz. Die getrockneten Textilien wurden von neun Personen mit entsprechender Erfahrung in der Beurteilung der Weichheit von Textilien auf ihren weichen Griff hin überprüft und im Vergleich einmal zu nicht mit Weichspülmitteln und zum anderen mit einem handelsüblichen Weichspülmittel behandelten Textilien beurteilt. Die Beurteilung erfolgt nach einem abgestuften Punktesystem, wobei die Endbeurteilung durch den arithmetischen Mittelwert wiedergegeben wird. Nach der Trocknung weist das so behandelte Textilgut einen ausgezeichneten weichen flauschigen Griff und ein im Vergleich zu handelsüblichen Mitteln stark verbessertes Rücknetzvermögen auf.

Das Rücknetzvermögen wird gemessen in Anlehnung an DIN 53 924 mit der Abänderung, daß die Streifen der Gewebe (Probestücke) 1,5 cm breit sind.

Neben der Verwendung als Weichspülmittel können die erfindungsgemäßen Verbindungen mit Erfolg in Haar- und Körperreinigungsmitteln, in Haushalts- und Industriereinigungsmitteln sowie in Textilhilfsmitteln wie insbesondere als Weichmacher/Avivage-Mittel mit hydrophilierenden Eigenschaften und hoher Substantivität für die Endausrüstung von Textilien eingesetzt werden.

Die in den nachfolgenden Beispielen verwendeten Abkürzungen haben die folgende Bedeutung:

| | |
|---|---|
| SZ = | Säurezahl |
| GAZ = | Gesamtaminzahl |
| TAZ = | Tertiäre Aminzahl |
| Cat. $SO_3$ = | Kationenaktive Substanz. |

Diese Analysenwerte werden nach den folgenden auf diesem Gebiet allgemein üblichen Methoden bestimmt:

Säurezahl (SZ)

Die Säurezahl ist ein Maß für den Gehalt eines Fettes oder von technischen Fettsäuren an freien

Säuren und gibt die Milligramm Kaliumhydroxid an, die notwendig sind, um 1 Gramm Substanz zu neutralisieren.

Die Werte werden bestimmt nach der DGF-Einheitsmethode C-V4.

Gehalt an Kationaktiv-Substanz (Cat. $SO_3$)

Diese Methode dient zur Bestimmung des Gehalts an kationenaktiven Substanzen. Kationenaktive Substanzen sind hier langkettige Verbindungen, welche quaternäre Ammoniumgruppen enthalten. Der Gehalt wird angegeben in % quaternäre Verbindung, berechnet als $SO_3$-Equivalent mit einem Molgewicht von 80 g/mol.

Der Gehalt wird bestimmt durch eine Zweiphasentitration gemäß ISO-Norm 2871-1 und 2871-2 (1988 E).

Gehalt an Trockensubstanz

Der Gehalt wird bestimmt durch Erhitzen und Trocknen bei 105 °C bis zur Gewichtskonstanz.

Gesamtaminzahl (GAZ), Tertiäraminzahl (TAZ)

Die Gesamtaminzahl gibt die Anzahl Milligramm Kaliumhydroxid an, die der Gesamtaminbasizität von 1 g der Aminverbindung equivalent sind (mg KOH/g). Die Tertiäraminzahl gibt die Anzahl Milligramm Kaliumhydroxid an, die der Tertiäraminbasizität von 1 g der Aminverbindung equivalent sind.

Die Werte werden bestimmt nach American Oil Chemists Society (A.O.C.S.) Official Method Tf 2a - 64.

Herstellung der erfindungsgemäßen Verbindungen

Beispiel 1

1150 g (2,03 mol; MG 566 g/mol) Dimerfettsäure D 96 werden mit 450 g (5,06 mol) Dimethylethanolamin versetzt und nach Zusatz von 2,3 g (0,2 %) hypophosphoriger Säure und 1,15 g (0,1 %) Methansulfonsäure unter $N_2$ auf 180 °C erwärmt. Das entstehende Reaktionswasser wird über eine Kolonne langsam abdestilliert. Nach Beendigung der Reaktion (ca. 12 h) wird das überschüssige Dimethylethanolamin im Vakuum bei 180 °C und 12 mbar abdestilliert.

| | | |
|---|---|---|
| Analysendaten: | Säurezahl | 2,8 |
| | GAZ | 148 |
| | TAZ | 147 |

Beispiel 2

386 g (0,5 mol) Dimerfettsäurediester gemäß Beispiel 1 werden mit 150 g Isopropanol gemischt und mit 147 g (1,17 mol) Dimethylsulfat bei 70 °C unter $N_2$ quaterniert, bis die GAZ kleiner als 4 ist.

| | | |
|---|---|---|
| Analysendaten: | TR | 80 % |
| | GAZ | 2,6 |
| | Cat. $SO_3$ sauer | 12,4 |
| | pH-Wert (5 %) | 6,9 |

Beispiel 3

Eine Lösung von 465 g (0,61 mol) Dimerfettsäurediester gemäß Beispiel 1 in 50 g Isopropanol werden durch langsames Zutropfen mit 121 g (1,2 mol) Milchsäure 90 % bei 50 °C unter gutem Rühren und unter $N_2$ neutralisiert.

| | | |
|---|---|---|
| Analysendaten: | TR<br>GAZ<br>pH-Wert (5 %) | 82,8 %<br>108<br>6,5 |

Beispiel 4

Eine Lösung von 850 g (1,1 mol) Dimerfettsäurediester gemäß Beispiel 1 in 110 g Isopropanol werden bei 60 °C mit 138 g (1,1 mol) Dimethylsulfat unter $N_2$ quaterniert. Anschließend wird die Mischung durch langsames Zutropfen mit 114 g (1,1 mol) Milchsäure 90 % unter gutem Rühren und unter $N_2$ neutralisiert.

| | | |
|---|---|---|
| Analysendaten: | TR<br>GAZ<br>Cat. $SO_3$ sauer<br>pH-Wert (5 %) | 87 %<br>53<br>14,9<br>6,3 |

Beispiel 5

1530 g (2,64 mol; MG 580 g/mol) Dimerfettsäure D 96 werden nach Zusatz von 3 g (0,2 %) hypophosphoriger Säure mit 650 g (6,4 mol) Dimethylaminopropylamin versetzt und unter $N_2$ auf 180 °C erhitzt. Das entstehende Reaktionswasser wird über eine Kolonne langsam abdestilliert. Nach Beendigung der Reaktion (ca. 4 h) wird der Aminüberschuß im Vakuum bei 180 °C und 12 mbar abdestilliert.

| | | |
|---|---|---|
| Analysendaten: | Säurezahl<br>GAZ<br>TAZ | 0,7<br>157<br>156 |

Beispiel 6

500 g (0,66 mol; MG 748 g/mol) Dimerfettsäurediamid gemäß Beispiel 5 werden nach Zusatz von 165 g 1,2-Propylenglykol unter $N_2$ bei 70 °C mit 171 g (1,35 mol) Dimethylsulfat quaterniert, bis die GAZ <3 ist.

| | | |
|---|---|---|
| Analysendaten: | TR<br>GAZ<br>Cat. $SO_3$ sauer<br>pH-Wert (5 %) | 82,2 %<br>1,5<br>12,0<br>7,2 |

Beispiel 7

Eine Lösung von 810 g (1,1 mol) Dimerfettsäurediamid gemäß Beispiel 5 in 250 g 1,2-Propylenglykol werden bei 60 °C mit 143 g (1,1 mol) Dimethylsulfat unter $N_2$ quaterniert. Anschließend wird durch langsames Zutropfen mit 113 g (1,1 mol) Milchsäure 90 % unter gutem Rühren und unter $N_2$ neutralisiert.

| | | |
|---|---|---|
| Analysendaten: | TR<br>GAZ<br>Cat. $SO_3$ sauer<br>pH-Wert (5 %) | 79 %<br>47<br>13<br>7,3 |

Beispiel 8

Eine Lösung von 530 g (0,75 mol; MG 714 g/mol) Dimerfettsäurediamid gemäß Beispiel 5 in 170 g 1,2-Propylenglykol werden mit 150 g (1,5 mol) Milchsäure 90 % bei 50 °C unter $N_2$ neutralisiert.

| | | |
|---|---|---|
| Analysendaten: | TR<br>GAZ<br>pH-Wert (5 %) | 71,2 %<br>95<br>6,1 |

Beispiel 9

Zu 540 g (0,75 mol; MG 725 g/mol) Dimerfettsäurediester gemäß Beispiel 1 werden bei 180 °C unter $N_2$ innerhalb von 2 h 76,5 g (0,75 mol) Dimethylaminopropylamin langsam getropft. Nach weiteren 4 h wird das freigesetzte Dimethylethanolamin im Vakuum bei 12 mbar und 180 °C abdestilliert.

| | | |
|---|---|---|
| Analysendaten: | SZ<br>GAZ | 2,0<br>151 |

Beispiel 10

740 g (1,0 mol; MG 740 g/mol) Dimerfettsäureesteramid gemäß Beispiel 9 werden nach Zusatz von 170 g Isopropanol unter $N_2$ bei 70 °C mit 249 g (1,94 mol) Dimethylsulfat quaterniert.

| | | |
|---|---|---|
| Analysendaten: | TR<br>GAZ<br>Cat. $SO_3$<br>pH-Wert (5 %) | 85 %<br>1,8<br>13,2<br>6,6 |

Beispiel 11

Eine Lösung von 740 g (1 mol) Dimerfettsäureesteramid gemäß Beispiel 9 in 80 g Isopropanol werden durch langsames Zutropfen mit 200 g (2 mol) Milchsäure 90 % bei 50 °C unter gutem Rühren und unter $N_2$ neutralisiert.

| | | |
|---|---|---|
| Analysendaten: | TR<br>GAZ<br>pH-Wert (5 %) | 85,2 %<br>112<br>5,3 |

Beispiel 12

Eine Lösung von 740 g (1 mol) Dimerfettsäureesteramid gemäß Beispiel 9 in 160 g Isopropanol werden bei 60 °C mit 126 g (1 mol) Dimethylsulfat unter $N_2$ quaterniert. Anschließend wird die Mischung durch langsames Zutropfen mit 100 g (1 mol) Milchsäure 90 % unter gutem Rühren und unter $N_2$ neutralisiert.

| | | |
|---|---|---|
| Analysendaten: | TR<br>GAZ<br>Cat. $SO_3$ sauer | 83 %<br>50<br>13,3 |

Anwendungstechnische Überprüfung

Die anwendungstechnische Überprüfung wurde durchgeführt nach den auf diesem Gebiet üblichen Testmethoden wie im folgenden angegeben:

Herstellung des Wäscheweichspülmittels

Die in der nachfolgenden Tabelle aufgeführten Beispiele wurden wie folgt hergestellt:
In das Leitungswasser von 9 °dH (deutsche Härtegrade) und 20 °C wurden unter gutem Rühren 0,625 g SANDOLAN[R] Walkblau NBL-150 1 % (Fa. Sandoz), 0,250 g DOW Antifoam DB-110A (der Fa. DOW Chemicals) eingerührt und danach die in den Beispielen angegebene Menge an erfindungsgemäßer Substanz gelöst. Danach wurde, soweit erforderlich, mit HCl bei den quaternären Verbindungen und mit Milchsäure bei den Salzen auf pH-Werte von ca. 3 eingestellt.

Überprüfung auf Rücknetzvermögen und Weichgriff

| | |
|---|---|
| Testgewebe: | Standard-Walkfrottiergewebe<br>100 % Baumwolle<br>Größe: 80 x 50 cm<br>Fa. Möwe, Reutlingen |

3 kg des Testgewebes wurden 2mal mit je 100 g Waschmittel (Testwaschmittel WFK-Testgewebe GmbH Krefeld) und anschließend 2mal ohne Waschmittel gewaschen (jeweils 95 °C-Waschprogramm mit Vorwäsche).

Die bei Raumtemperatur getrockneten Testgewebe wurden anschließend in die Weichspüllösung, enthaltend 0,025 Gew.-% erfindungsgemäße Verbindung bzw. handelsübliche Weichspülmittel, in Leitungswasser von 9 °dH 15 - 20 °C 10 min eingetaucht.

Die bei Raumtemperatur getrockneten Gewebe wurden von 9 Testpersonen auf ihren Weichgriff beurteilt. Als Bewertung wurden Punkte von 0 - 5 vergeben, wobei 0 = hart und 5 = sehr weich = gut bedeutet. Die Endbeurteilung ist der arithmetische Mittelwert.

Das Rücknetzvermögen wurde gemessen in Anlehnung an DIN 53 924 mit der Abänderung, daß die Streifen der Probestücke 1,5 cm breit sind.

Wäscheweichspülmittel

| Beispiel | Erfindungsgemäße Komponente gem. Beispiel | Erfindungsgemäße Komponente Menge 100 g Gesamtmischung | Beurteilung | Rücknetzvermögen | Weichgriff |
|---|---|---|---|---|---|
| 1 | 2 | 31 g | klare, niedrigviskose Lösung | 100 % | gut |
| 2 | 2 | 6,3 g | klare niedrigviskose Lösung | 100 % | gut |
| 3 | 4 | 30,5 g | klare, niedrigviskose Lösung | 100 % | gut |
| 4 | 4 | 6 g | klare, niedrigviskose Lösung | 100 % | gut |
| 5 | 3 | 24,2 g | klare, niedrigviskose Lösung | 100 % | gut |
| 6 | 6 | 31 g | klare, niedrigviskose Lösung | 97 % | gut |
| 7 | 6 | 6 g | klare, niedrigviskose Lösung | 97 % | gut |
| 8 | 7 | 38 g | klare, niedrigviskose Lösung | 98 % | gut |
| 9 | 7 | 18,8 g | klare, niedrigviskose Lösung | 98 % | gut |
| 10 | 8 | 35,1 g | klare, niedrigviskose Lösung | 97 % | gut |
| 11 | 8 | 7 g | klare, niedrigviskose Lösung | 97 % | gut |
| 12[a] | 10 | 29,3 g | klare Lösung | 97 % | gut |
| 13[a] | 10 | 6 g | klare, niedrigviskose Lösung | 97 % | gut |

| Beispiel | Erfindungsgemäße Komponente: gem.Beispiel | Erfindungsgemäße Komponente: Menge 100 g Gesamtmischung | Beurteilung | Rücknetzvermögen | Weichgriff |
|---|---|---|---|---|---|
| 14 | 12 | 28,5 g | klare, niedrigviskose Lösung | 100 % | gut |
| 15 | 12 | 5,7 g | klare, niedrigviskose Lösung | 100 % | gut |
| 16 | 11 | 28,4 g | klare, niedrigviskose Lösung | 99 % | gut |
| 17 | 11 | 5,7 g | klare, niedrigviskose Lösung | 99 % | gut |
| 18 | 2 | 37,1 g | klare, niedrigviskose Lösung | 100 % | gut |
| 19 | 6 | 37,1 g | klare, niedrigviskose Lösung | 97 % | gut |
| 20 | 7 | (30 %) | klare, dünnflüssige Lösung | 98 % | gut |
| Vergleichsversuche | | | | | |
| 1 | Vergleich[b)] | 18,8 g | Dispersion | 77 % | gut |
| 2 | " | 25,1 g | Dispersion | 77 % | gut |
| 3 | Vergleich[c)] | 6,7 g | Dispersion | 64 % | sehr gut |
| 4 | - | - | - | 100 % | unbefriedigend |

[a] = bei 40 °C Wassertemperatur die auf 50 °C vorgewärmte erfindungsgemäße Komponente eingearbeitet

[b)] = Vergleichsversuch: Handelsübliche quaternäre Ammoniumverbindung (3-Alkoxyloxy-2-hydroxypropyl)-trimethylammonium-chlorid.

[c)] = REWOQUAT[R] W 90: Quaternäres Imidazoliniumderivat der Fa. REWO Chemische Werke, Steinau an der Straße.

Die erfindungsgemäßen Verbindungen lassen sich mit gutem Erfolg auch in kosmetischen Rezepturen verwenden, wobei sie auf dem Haarpflegesektor je nach Rezeptur sowohl zur Reinigung, das heißt als Shampoo, als auch zur Pflege als Haarnachspülung zur Verbesserung der Kämmbarkeit eingesetzt werden können.

Im Einsatz in Shampoos haben sie gegenüber herkömmlichen kationischen Verbindungen den großen Vorteil, daß sie sich in Wasser auch in Gegenwart von anionischen Tensiden klar lösen lassen, während die Produkte gemäß Stand der Technik nur Dispersionen ergeben. Der Vorteil von Lösungen gegenüber Dispersionen hinsichtlich der Lagerstabilität über einen großen Temperaturbereich ist bekannt.

Ein weiterer Vorteil liegt darin, daß bei der gleichzeitigen Anwesenheit von anionischen und kationischen Verbindungen in einer Rezeptur diese sowohl reinigende als auch pflegende Wirkung entfaltet.

Außerdem kommen diese Rezepturen der modernen Auffassung nach einem mehrseitig anwendbaren Mittel entgegen, da sie außer zur Haarreinigung und gleichzeitiger Haarpflege auch reinigend und pflegend auf die Haut wirken. Bei Mitverwendung der erfindungsgemäßen Verbindungen lassen sich also die Forderungen der Praxis nach Body-Hair-Care-Shampoos problemlos erfüllen.

Für die kosmetischen Anwendungen können die auf diesen Gebieten üblichen Tenside, Duftstoffe, Konservierungsmittel, Farbstoffe, Pflanzenextrakte bzw. sonstige kosmetische Additive mitverwendet werden.

Als Tenside kommen neben den bekannten Betainen, amphoteren und nichtionischen Verbindungen für die reinigenden Rezepturen insbesondere die anionischen Tenside wie Carboxylate, Alkylsulfate, Alkylethersulfate, Alkylsulfonate, Alkylethersulfonate, Alkylsulfosuccinate in Betracht. Erfindungsgemäß bevorzugt werden Alkylamidobetaine wie REWOTERIC[R] [1)] AM B 13 und AM B 14, Carboxyglycinate wie REWOTERIC[R] AM 2 C NM, Carboxypropionate wie REWOTERIC[R] AM KSF 40, Sulfobetaine wie REWOTERIC[R] AM CAS, anionische Tenside wie Ethersulfate REWOPOL[R] [2)] NL 3, Ethercarboxylate wie REWOPOL[R] CLN 100, Sulfosuccinate wie REWOPOL[R] SB FA 30, REWOPOL[R] SBZ, REWODERM[R] [3)] SPS, nichtionische Tenside wie Glycerinfettsäureesterethoxylate wie REWODERM[R] ES 90, Glycerinmonostearat wie REWOMUL[R] [4)] MG, Cetylalkohol ([1), 2), 3), 4)] = Warenzeichen der Fa. REWO Chemische Werke GmbH, Steinau an der Straße).

Neben den erfindungsgemäßen Verbindungen, welche in Mengen von 0,5 - 10 Gew.-Teilen, insbesondere 1 - 8 Gew.-Teilen und vorzugsweise 1 - 3 Gew.-Teilen, bei Shampoos und 1 - 7 Gew.-Teilen, vorzugsweise 1 - 5 Gew.-Teilen, bei Haarnachspülmitteln bzw. Haarkurpackungen eingesetzt werden, werden die anderen Tenside bei den Shampoos üblicherweise in Mengen von 1 - 20 Gew.-Teilen, vorzugsweise 5 - 15 Gew.-Teilen, bei den Haarnachspülmitteln bzw. Haarkurpackungen in Mengen von 0,1 - 10 Gew.-Teilen, vorzugsweise 1 - 5 Gew.-Teilen, mitverwendet.

Als Verdickungsmittel werden 1 - 8 Gew.-Teile der auf diesem Gebiet üblichen Verbindungen wie Glycerinfettsäureesterethoxylate, Fettalkoholethoxylate, Fettsäurealkylolamide und die üblichen Alkali-, Erdalkali- und Ammoniumsalze, welche bei 20 °C in Mengen von mindestens 1 Gew.-% in Wasser löslich sind, wie insbesondere NaCl, $NH_4Cl$ eingesetzt.

## Kosmetische Formulierungen

Die nachfolgenden Shampoos für Körper und Haar können durch einfaches Einmischen der Rezepturbestandteile in Wasser hergestellt werden.

Rezeptur Nr. 1: Conditioning Shampoo

50 Teile REWOPOL$^R$ NL 3-28[1)]
10 Teile REWOTERIC$^R$ AM KSF 40[2)]
2 Teile REWOMINOX$^R$ B 204[3)]
20 Teile erfindungsgemäße Beispiele 6 und 10
0,3 Teile Citronensäure
3,0 Teile Natriumchlorid
ad 100 Teile Wasser. demin. $^R$ = Warenzeichen der Fa. REWO Chem. Werke GmbH

Rezeptur Nr. 2: Conditioning Shampoo

50 Teile REWOPOL$^R$ NL 3-28[1)]
10 Teile REWOPOL$^R$ SB FA 30[4)]
2 Teile REWOMINOX$^R$ B 204[3)]
1,3 Teile erfindungsgemäße Beispiele 6 und 10
0,1 Teile Citronensäure
4,0 Teile Natriumchlorid
ad 100 Teile Wasser, demin.

Rezeptur Nr. 6: Dusch- und Haarshampoo mit conditionierender Wirkung

1,5 Teile erfindungsgemäßes Beispiel 2
2 Teile REWODERM$^R$ ES 90[6)]
2 Teile REWODERM$^R$ LIS 75[10)]
10 Teile REWOTERIC$^R$ AM B 13[11)]
25 Teile REWOPOL$^{R)}$ NL 3-28[1)]
8 Teile REWOPOL$^{R)}$ SBZ[12)]
ad 100 Teile demin. Wasser

Alle Rezepturen wurden auf Verbesserung der Kämmbarkeit der Haare überprüft und Rezeptur 6 zusätzlich auf das Hautglättegefühl nach dem Duschen. Die Tests wurden durchgeführt von 10 auf diesem Gebiet erfahrenen Prüfern, wobei der statistische Mittelwert zur Endbewertung herangezogen wurde.

Die Kämmbarkeit wurde bei allen Rezepturen als gut bei reduziertem Kämmwiderstand beurteilt. Auf

[1)] = Natriumlaurylethersulfat

[2)] = amphoteres Tensid, salzfrei

[3)] = Alkylamidopropyldimethylaminoxid

[1)] = Natriumlaurylethersulfat

[4)] = Fettalkoholpolyglycolethersulfosuccinat

[3)] = Alkylamidopropyldimethylaminoxid

[6)] = Fettsäuremonoglyceridpolyglykolether

[10)] = Fettsäuremonoglyceridpolyglykolether, modif.

[11)] = Alkylamidobetain

[12)] = Fettsäurepolyglykolestersulfosuccinat.

der von 1 - 7 reichenden Beurteilungsskala wurden durchschnittliche Werte von 4 - 7 erzielt.

Das Kautgefühl auf der nassen Haut wird bei Rezeptur 6 als cremig weich, bei der trockenen Haut als glatt weich beurteilt.

Die nachfolgenden Rezepturen wurden wie folgt hergestellt:

Rezeptur Nr. 3: Haarnachspülmittel, kalt herstellbar

1,0 Teile NATROSOL[R 1)]250 HHR[7)]
1,0 Teile REWOQUAT[R] RTM 50[5)]
2,0 Teile erfindungsgemäße Beispiele 6 und 10
1,0 Teile REWODERM[R] ES 90[6)]
0,4 Teile Citronensäure
ad 100 Teile Wasser, demin.

Rezeptur Nr. 4: Haarkurpackung

| | |
|---|---|
| Phase A: | 5,5 Teile erfindungsgemäße Beispiele 6 und 10 |
| | 1,5 Teile Cetylalkohol |
| | 3,0 Teile REWOMUL[R] MG[8)] |
| Phase B: | 2,0 Teile REWODERM[R] ES 90[6)] |
| | 2,0 Teile REWOPOL[R] SB FA 30[4)] |
| | 0,2 Teile Citronensäure |
| | 85,8 Teile Wasser,demin. |

Rezeptur Nr. 5: Haarnachspülung

| | |
|---|---|
| Phase A: | 3,0 Teile erfindungsgemäßes Beispiel 10 |
| | 1,0 Teile Cetylalkohol |
| | 1,0 Teile TEGINACID[R 2) 9)] |
| Phase B: | 0,2 Teile Citronensäure |
| | ad 100 Teile Wasser, demin. |

Das Haarnachspülmittel der Rezeptur 3 wird hergestellt durch Vorquellen von NATROSOL[R] 250 HHR in demineralisiertem Wasser bei Raumtemperatur und anschließender Zugabe der restlichen Komponenten unter intensivem Rühren. Es wird eine klare mittelviskose lagerstabile Lösung erhalten.

Das Haarnachspülmittel der Rezeptur 5 und die Haarkurpackung der Rezeptur 4 werden hergestellt, indem vorab zwei Phasen A) und B) separat durch jeweiliges Zusammenmischen unter Erwärmen auf 65

[R 1)] = eingetragenes Warenzeichen der Fa. Aqualon

[7)] = Hydroxyethylcellulose

[5)] = Rizinolsäurepropylamidotrimethylammoniummethosulfat

[6)] = Fettsäuremonoglyceridpolyglykolether

[8)] = Clycerinmonostearat

[6)] = Fettsäuremonoglyceridpolyglykolether

[4)] = Fettalkoholpolyglycolethersulfosuccinat

[R 2)] = eingetragenes Warenzeichen der Fa. Goldschmidt

[9)] = Glycerinmonostearat, säurestabil.

° C bei gleichzeitigem guten Rühren hergestellt werden und anschließend Phase A) in Phase B) eindispergiert.

Diese Rezepturen wurden wie oben angegeben auf Naß- und Trocken-Kämmbarkeit überprüft.

Sowohl die Naß- als auch die Trockenkämmbarkeit wurde von der Mehrzahl der Prüfer in der bis 10 reichenden Beurteilungsskala mit der maximal erreichbaren Punktzahl von 10 beurteilt. Der statistische Mittelwert lag bei 8.

| | |
|---|---|
| Bewertungsskala: | 1 - 3 schlecht durchkämmbar<br>4 - 7 gut durchkämmbar, kaum Kämmwiderstand<br>8 -10 sehr gut durchkämmbar, weiter verringerter Kämmwiderstand. |

**Patentansprüche**

**1.** Ammoniumverbindungen der allgemeinen Formel (1)

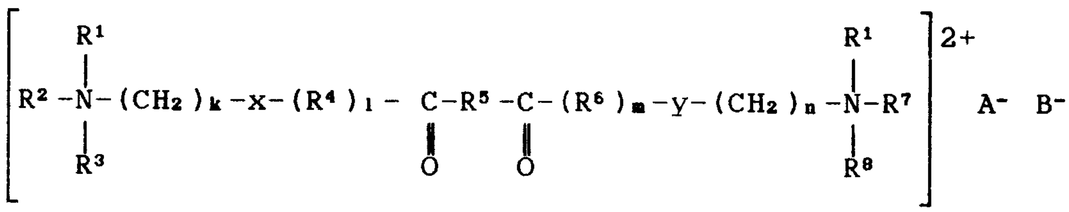

(1)

mit der Bedeutung von

| | |
|---|---|
| $R^1$ | = gleich oder verschieden H, $-CH_3$, $-C_2H_5$, $-(C_3H_5O)_{C1}H$, $-(C_2H_4O)_{C2}H$ |
| $R^2$, $R^3$, $R^7$, $R^8$ | = gleich oder verschieden ein geradkettiger verzweigter Alkyl-oder Alkylenrest mit 1 - 18 C-Atomen |
| $R^4$, $R^6$ | = $-(CH_2\text{-}CHR^9\text{-}O)_a\text{-}(CH_2\text{-}CHR^{10}\text{-}O)_b\text{-}$ mit $R^9 R^{10}$ = H oder $-CH_3$, a, b = gleich oder verschieden 0 - 10 |
| $R^5$ | = Rest der dimerisierten Fettsäure |
| x, y | = O und/oder NH |
| $A^-B^-$ | = gleich oder verschieden das Anion einer wasserlöslichen ein- oder mehrwertigen anorganischen oder organischen Säure wie Methylschwefelsäure, Ethylschwefelsäure, einer Halogenwasserstoffsäure, Phosphorsäure, Ameisensäure, Essigsäure, Oxalsäure, Glykolsäure, Zitronensäure, Weinsäure, Äpfelsäure und insbesondere Salzsäure und Milchsäure sein können und die Gesamtzahl oder negativen Ladungen im Molekül gleich der positiven ist |
| k, n | = gleich oder verschieden 2 - 4 |
| $C_1$, $C_2$ | = gleich oder verschieden 1 - 5 |
| l, m | = gleich oder verschieden 0 oder 1, mit l = O, wenn x = NH und m = O wenn y = NH |

**2.** Ammoniumverbindungen gemäß Anspruch 1, worin
$R^1$ gleich oder verschieden H oder $-CH_3$ ist, $R^{2,3,7,8}$ der Methylrest,
$R^5$ der Rest der dimerisierten Fettsäure mit einem Dimergehalt von ≧80 Gew.-% ist,
k, n = 2 oder 3, l und m = 0 und $A^-$und $B^-$ gleich oder verschieden der Lactatrest oder der Methosulfatrest ist.

**3.** Wässrige Weichspülmittel, enthaltend
A) 5 bis 30 Gewichts-% mindestens einer der Verbindungen der allgemeinen Formel 1

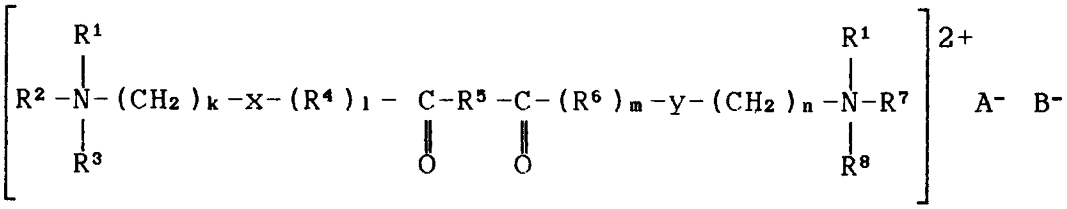

(1)

mit der Bedeutung von

| | | |
|---|---|---|
| $R^1$ | = | gleich oder verschieden H/-$CH_3$, -$C_2H_5$ -$(C_3H_5O)_{C1}H$, -$(C_2H_4O)_{C2}H$ |
| $R^2$, $R^3$, $R^7$, $R^8$ | = | gleich oder verschieden ein geradkettiger verzweigter Alkyl-oder Alkylenrest mit 1 - 18 C-Atomen |
| $R^4$, $R^6$ | = | -$(CH_2$-$CHR^9$-$O)_a$-$(CH_2$-$CHR^{10}$-$O)_b$- mit $R^9 R^{10}$ = H oder -$CH_3$, a, b = gleich oder verschieden 0 - 10 |
| $R^5$ | = | Rest der dimerisierten Fettsäure |
| x, y | = | O und/oder NH |
| $A^-B^-$ | = | gleich oder verschieden das Anion einer wasserlöslichen ein- oder mehrwertigen anorganischen oder organischen Säure wie Methylschwefelsäure, Ethylschwefelsäure, einer Halogenwasserstoffsäure, Phosphorsäure, Ameisensäure, Essigsäure, Oxalsäure, Glykolsäure, Zitronensäure, Weinsäure, Äpfelsäure und insbesondere Salzsäure und Milchsäure sein können und die Gesamtzahl oder negativen Ladungen im Molekül gleich der positiven ist |
| k, n | = | gleich oder verschieden 2 - 4 |
| $C_1$, $C_2$ | = | gleich oder verschieden 1 - 5 |
| l, m | = | gleich oder verschieden 0 oder 1, mit l = O, wenn x = NH und m = O wenn y = NH |

und ergänzend auf 100 Gew.-%

B) Wasser, Farbstoffe, Parfüm, Verdickungsmittel und übliche Hilfs- und Zusatzstoffe.

**4.** Wässrige Weichspülmittel gemäß Anspruch 3, dadurch gekennzeichnet, daß in der allgemeinen Formel 1, worin
$R^1$ gleich oder verschieden H oder -$CH_3$ ist, $R^{2,3,7,8}$ der Methylrest,
$R^5$ der Rest der dimerisierten Fettsäure mit einem Dimergehalt von ≧80 Gew.-% ist,
k, n = 2 oder 3, l und m = 0 und $A^-$und $B^-$ gleich oder verschieden der Lactatrest oder der Methosulfatrest ist.

**5.** Verfahren zur Herstellung der Verbindungen der allgemeinen Formel (1)

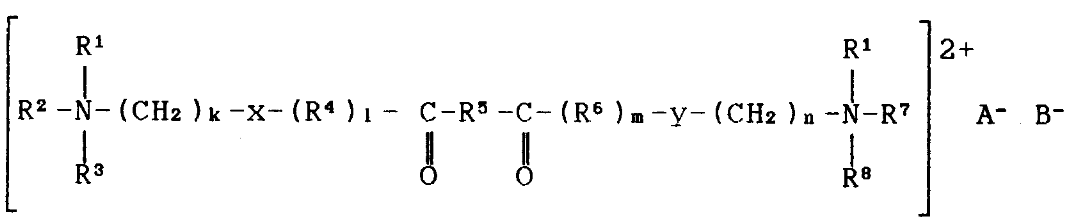

(1)

worin $R^1$-$R^8$, x, y, $A^-$, $B^-$, k, l, m, n die oben angegebene Bedeutung haben, wobei das prim./tert. Amin oder das Alkanolamin in Gegenwart saurer Katalysatoren wie Methansulfonsäure oder hypophosphoriger Säure unter Stickstoff bei Temperaturen von 140 - 200 °C verestert bzw. amidiert und das entstandene Kondensat kontinuierlich, gegebenenfalls im Endstadium bei Unterdruck, abdestilliert wird.

**6.** Wässrige Haarreinigungs- und Pflegemittel, enthaltend

a) 0,5 - 10 Gew.-Teile mindestens eine der Verbindungen der allgemeinen Formel (1)
b) 1,0 - 20 Gew.-Teile mindestens eines nichtionischen, amphoteren, zwitterionischen, ionischen Tensids
c) 0,1 - 10 Gew.-Teile Verdickungsmittel, Duftstoffe, Konservierungsmittel, Farbstoffe, Pflanzenextrakte und sonstige Zusatz- und Hilfsstoffe und
d) ad 100 Wasser.

**7.** Wässriges Dusch- und Haarshampoo, enthaltend
a) 0,5 - 10 Gew.-Teile der Verbindungen der allgemeinen Formel (1), worin $R^5$ der Rest der dimerisierten Fettsäure mit einem Dimergehalt von ≧80 Gew.-% ist, k, n = 2 oder 3, l und m = 0 und $A^-$und $B^-$ gleich oder verschieden der Lactatrest oder der Methosulfatrest ist,
b) 1,0 - 10 Gew.-Teile mindestens eines nichtionischen, amphoteren, zwitterionischen, ionischen Tensids
c) 0,1 - 5 Gew.-Teile Verdickungsmittel, Duftstoffe, Konservierungsmittel, Farbstoffe, Pflanzenextrakte und sonstige Zusatz- und Hilfsstoffe und
d) ad 100 Wasser.

**8.** Wässriges Haarconditionsierungsmittel, enthaltend
a) 1 - 7 Gew.-Teile der Verbindungen der allgemeinen Formel (1), worin
$R^5$ der Rest der dimerisierten Fettsäure mit einem Dimergehalt von ≧80 Gew.-% ist,
k, n = 2 oder 3, l und m = 0 und $A^-$und $B^-$ gleich oder verschieden der Lactatrest oder der Methosulfatrest ist,
b) 0,1 - 20 Gew.-Teile mindestens eines nichtionischen, amphoteren zwitterionischen, ionischen Tensids
c) 0,1 - 5 Gew.-Teile Verdickungsmittel, Duftstoffe, Konservierungsmittel, Farbstoffe, Pflanzenextrakte und sonstige Zusatz- und Hilfsstoffe und
d) ad 100 Wasser.

**Patentansprüche für folgenden Vertragsstaat : ES**

**1.** Verfahren zur Herstellung von Ammoniumverbindungen der allgemeinen Formel (1)

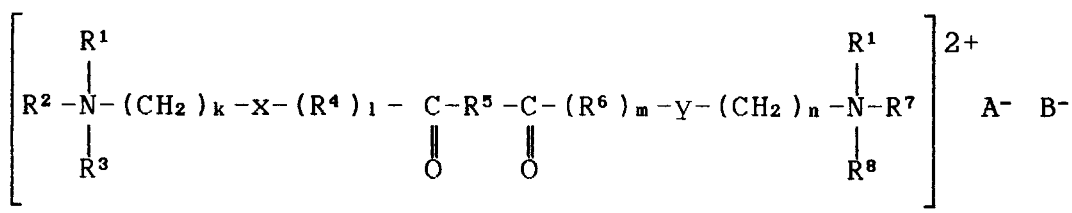

(1)

mit der Bedeutung von

| | |
|---|---|
| $R^1$ | = gleich oder verschieden H, $-CH_3$, $-C_2H_5$, $-(C_3H_5O)_{C1}H$, $-(C_2H_4O)_{C2}H$ |
| $R^2$, $R^3$, $R^7$, $R^8$ | = gleich oder verschieden ein geradkettiger verzweigter Alkyl-oder Alkylenrest mit 1 - 18 C-Atomen |
| $R^4$, $R^6$ | = $-(CH_2-CHR^9-O)_a-(CH_2-CHR^{10}-O)_b-$ mit $R^9 R^{10}$ = H oder $-CH_3$, a, b = gleich oder verschieden 0 - 10 |
| $R^5$ | = Rest der dimerisierten Fettsäure |
| x, y | = O und/oder NH |
| $A^-B^-$ | = gleich oder verschieden das Anion einer wasserlöslichen ein- oder mehrwertigen anorganischen oder organischen Säure wie Methylschwefelsäure, Ethylschwefelsäure, einer Halogenwasserstoffsäure, Phosphorsäure, Ameisensäure, Essigsäure, Oxalsäure, Glykolsäure, Zitronensäure, Weinsäure, Äpfelsäure und insbesondere Salzsäure und Milchsäure sein können und die Gesamtzahl oder negativen Ladungen im Molekül gleich der positiven ist |
| k, n | = gleich oder verschieden 2 - 4 |
| $C_1$, $C_2$ | = gleich oder verschieden 1 - 5 |

l, m = gleich oder verschieden 0 oder 1, mit l = O, wenn x = NH und m = O wenn y = NH,

wobei das prim./tert. Amin oder das Alkanolamin in Gegenwart saurer Katalysatoren wie Methansulfonsäure oder hypophosphoriger Säure unter Stickstoff bei Temperaturen von 140 - 200 °C verestert bzw. amidiert und das entstandene Kondensat kontinuierlich, gegebenenfalls im Endstadium bei Unterdruck, abdestilliert wird.

**2.** Ammoniumverbindungen gemäß Anspruch 1, worin
$R^1$ gleich oder verschieden H oder $-CH_3$ ist, $R^{2,3,7,8}$ der Methylrest,
$R^5$ der Rest der dimerisierten Fettsäure mit einem Dimergehalt von ≧80 Gew.-% ist,
k, n = 2 oder 3, l und m = 0 und $A^-$und $B^-$ gleich oder verschieden der Lactatrest oder der Methosulfatrest ist.

**3.** Wässrige Weichspülmittel, enthaltend
A) 5 bis 30 Gewichts-% mindestens einer der Verbindungen der allgemeinen Formel 1

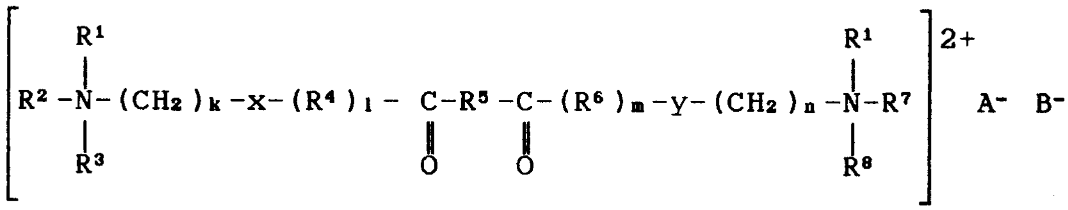

(1)

mit der Bedeutung von

| | |
|---|---|
| $R^1$ | = gleich oder verschieden $H/-CH_3$, $-C_2H_5$ $-(C_3H_5O)_{C1}H$, $-(C_2H_4O)_{C2}H$ |
| $R^2$, $R^3$, $R^7$, $R^8$ | = gleich oder verschieden ein geradkettiger verzweigter Alkyl-oder Alkylenrest mit 1 - 18 C-Atomen |
| $R^4$, $R^6$ | = $-(CH_2-CHR^9-O)_a-(CH_2-CHR^{10}-O)_b-$ mit $R^9R^{10}$ = H oder $-CH_3$, a, b = gleich oder verschieden 0 - 10 |
| $R^5$ | = Rest der dimerisierten Fettsäure |
| x, y | = O und/oder NH |
| $A^-B^-$ | = gleich oder verschieden das Anion einer wasserlöslichen ein- oder mehrwertigen anorganischen oder organischen Säure wie Methylschwefelsäure, Ethylschwefelsäure, einer Halogenwasserstoffsäure, Phosphorsäure, Ameisensäure, Essigsäure, Oxalsäure, Glykolsäure, Zitronensäure, Weinsäure, Äpfelsäure und insbesondere Salzsäure und Milchsäure sein können und die Gesamtzahl oder negativen Ladungen im Molekül gleich der positiven ist |
| k, n | = gleich oder verschieden 2 - 4 |
| $C_1$, $C_2$ | = gleich oder verschieden 1 - 5 |
| l, m | = gleich oder verschieden 0 oder 1, mit l = O, wenn x = NH und m = 0 wenn y = NH |

und ergänzend auf 100 Gew.-%
B) Wasser, Farbstoffe, Parfüm, Verdickungsmittel und übliche Hilfs- und Zusatzstoffe.

**4.** Wässrige Weichspülmittel gemäß Anspruch 3, dadurch gekennzeichnet, daß in der allgemeinen Formel 1, worin
$R^1$ gleich oder verschieden H oder $-CH_3$ ist, $R^{2,3,7,8}$ der Methylrest,
$R^5$ der Rest der dimerisierten Fettsäure mit einem Dimergehalt von ≧80 Gew.-% ist,
k, n = 2 oder 3, l und m = 0 und $A^-$und $B^-$ gleich oder verschieden der Lactatrest oder der Methosulfatrest ist.

**5.** Wässrige Haarreinigungs- und Pflegemittel, enthaltend
a) 0,5 - 10 Gew.-Teile mindestens eine der Verbindungen der allgemeinen Formel (1)
b) 1,0 - 20 Gew.-Teile mindestens eines nichtionischen, amphoteren, zwitterionischen, ionischen Tensids

c) 0,1 - 10 Gew.-Teile Verdickungsmittel, Duftstoffe, Konservierungsmittel, Farbstoffe, Pflanzenextrakte und sonstige Zusatz- und Hilfsstoffe und
d) ad 100 Wasser.

**6.** Wässriges Dusch- und Haarshampoo, enthaltend
a) 0,5 - 10 Gew.-Teile der Verbindungen der allgemeinen Formel (1), worin
$R^5$ der Rest der dimerisierten Fettsäure mit einem Dimergehalt von ≥80 Gew.-% ist,
k, n = 2 oder 3, l und m = 0 und $A^-$und $B^-$ gleich oder verschieden der Lactatrest oder der Methosulfatrest ist,
b) 1,0 - 10 Gew.-Teile mindestens eines nichtionischen, amphoteren, zwitterionischen, ionischen Tensids
c) 0,1 - 5 Gew.-Teile Verdickungsmittel, Duftstoffe, Konservierungsmittel, Farbstoffe, Pflanzenextrakte und sonstige Zusatz- und Hilfsstoffe und
d) ad 100 Wasser.

**7.** Wässriges Haarconditionsierungsmittel, enthaltend
a) 1 - 7 Gew.-Teile der Verbindungen der allgemeinen Formel (1), worin
$R^5$ der Rest der dimerisierten Fettsäure mit einem Dimergehalt von ≥80 Gew.-% ist,
k, n = 2 oder 3, l und m = 0 und $A^-$und $B^-$ gleich oder verschieden der Lactatrest oder der Methosulfatrest ist,
b) 0,1 - 20 Gew.-Teile mindestens eines nichtionischen, amphoteren zwitterionischen, ionischen Tensids
c) 0,1 - 5 Gew.-Teile Verdickungsmittel, Duftstoffe, Konservierungsmittel, Farbstoffe, Pflanzenextrakte und sonstige Zusatz- und Hilfsstoffe und
d) ad 100 Wasser.